# EUROPEAN PATENT SPECIFICATION

(11) **EP 0 357 270 B1**
(45) Date of publication and mention of the grant of the patent: **21.07.1993**
(21) Application number: 89308133.1
(22) Date of filing: 10.08.1989
(51) Int. Cl.: A61F 2/34, A61F 2/46, A61B 17/16

(54) **Rim-bearing acetabular component of hip joint prosthesis**
Mit einem Stützringflansch versehenes Acetabularteil einer Hüftgelenksprothese
Composant acétabulaire d'une prothèse de l'articulation de la hanche, muni d'une collerette d'appui annulaire

(30) Priority: 17.08.1988 GB 8819587
(43) Date of publication of application: 07.03.1990
(73) Proprietor: MINNESOTA MINING AND MANUFACTURING COMPANY, St. Paul, Minnesota 55101 (US)
(72) Inventor: Shelley, Philip, Dinnington S31 7YL (GB)
(74) Representative: Bowman, Paul Alan

(56) References cited:
- EP-A- 0 022 308
- EP-A- 0 118 194
- EP-A- 0 120 595
- CH-A- 663 893
- DE-A- 3 310 944
- DE-U- 8 701 242
- US-A- 4 180 873

## Description

This invention relates to a hip joint prosthesis and in particular to the acetabular component of a hip joint prosthesis.

Prosthesis for the replacement of hip joints are well known. Originally, only the ball-end on the head of the femur could be replaced but it has since proved possible to replace either part of the hip joint i.e. the acetabular socket of the joint or the ball-end on the femur.

Known acetabular cup implants, which form the socket portion of an artificial hip joint, comprise a metal cup shell, which is secured within a cavity in the pelvic bone of a patient, and an inner liner of plastics material which provides a spherical bearing surface for receiving the ball portion of the joint. The metal cup shell may be provided with an external thread to facilitate anchorage to the pelvic bone or may be secured by other means such as cement or screws.

Most current designs of metal cup shells can be grouped into two basic profiles - frusto-conical and hemispherical since these shapes may be conveniently fabricated by rotating reamers. In all cases the designs rely upon the floor and internal walls of the acetabulum for anchorage and to transmit the forces to which the joint is subjected.

There are several designs of acetabular cup implants which comprises a continuous or discontinuous circumferential flange around the opening of the cup implant. U.S. Patent No. 4563778 discloses an acetabular cup assembly having a circumferential flange, a cup assembly being adapted for securing with bone cement. U.S. Patent No. 3982281 discloses an acetabular cup assembly possessing a circumferential flange which cup assembly is inserted into the acetabulum so that there is initially clearance between the flange and the bone structure to allow settling movement in the device before the flange contacts the bone. U.S. Patent No. 4180873 discloses a frusto-conical cup shell possessing a circumferential flange which is force fitted into a prepared bone void of the acetabulum.

It is an anatomical fact that the thickest and strongest section of the pelvis in the acetabular region is the rim of the acetabulum. However, this rim presents an uneven and irregular margin which has precluded its use for load bearing in a hip joint prosthesis. It has now been found that by smoothing the rim of the acetabulum and providing a metal cup shell with a flange which bears on the machined surface the potential load bearing capacity of the rim of the acetabulum can be effectively utilized in addition to the internal surfaces of the acetabulum.

Therefore according to one aspect of the present invention there is provided an acetabular implant for a hip joint prosthesis comprising a metal cup shell adapted for securing in the acetabulum of a patient, the metal cup shell having a bore into which a liner may be inserted to provide a spherical bearing surface for the ball portion of the hip joint, characterised in that the metal cup shell comprises three apertures positioned for insertion of screws into the ilium, ischium and pubic ramus of the patient and a continuous circumferential flange around the opening of the bore, the flange having an outside diameter which is at least 10% greater than the outside diameter of the remainder of the metal cup shell at its widest point, the circumferential flange providing a bearing surface for contacting the rim of the acetabulum of the patient when the metal cup shell is fitted.

The circumferential flange of the metal cup shell of the invention provides an effective means of transmitting forces in the hip joint to the pelvic bone thereby reducing stresses in other parts of the joint. The circumferential flange generally has an outside diameter which is from 10 to 40% greater than that of the remainder of the metal cup shell, preferably 20 to 40% greater.

The bearing surface of the circumferential flange is preferably planar and at a right angle to the longitudinal axis of the bore although the plane of the bearing surface may be slightly inclined with respect to the longitudinal axis of the bore. The bearing surface may also be frusto-conical or curved. The flange is preferably continuous to provide maximum transmission of forces to the pelvic bone but some discontinuities are permissible e.g. slits or notches.

The metal cup shell is secured to the acetabulum by conventional techniques e.g. cement, screw thread on the outer surface or screws passing through the shell. In order to maximise the diameter and length of the screws (and the anchorage of the metal cup shell) the arrangement is to use three screws projecting into the ilium, ischium and pubic ramus. The metal cup shell has three apertures for accepting such screws drilled at the correct angles for optimum positioning and the screws may readily be inserted through a guide to ensure accurate positioning. The following configuration of apertures and positioning of the screws is particularly useful:
when viewed in plan the angle formed between a line passing through the centre of the aperture for the superior screw and the centre of the bore and a line passing through the centre of an aperture for an inferior screw and the centre of the bore is in the range 100 to 160^{o}; preferably the apertures are positioned at 0^{o}, 130^{o} and 230^{o};
in the side view the angle formed between the longitudinal axis of the bore and the longitudinal axis of the aperture for the superior screw and the longitudinal axis of the bore is from 20 to 45^{o} preferably 30^{o}, and the angle formed between the longitudinal axis of an aperture for an inferior screw and the longitudinal axis of the bore is from 35 to 75^{o}, preferably 50^{o}.

According to a further aspect of the invention there is provided for use in a hip joint prosthesis the combination of an acetabular implant of the invention and one or both of:
- a reaming tool comprising a blade for shaving the rim of the acetabulum, and
- a guide tool for aligning the screws used to anchor the acetabular implant in position with the apertures in the metal shell.

The invention will now be described with reference to the accompanying drawings in which:
- Figure 1 represents a cross-section through a metal shell cup in accordance with the invention,
- Figure 2 represents a plan view of a shell cup in accordance with the invention,
- Figure 3 represents a section through an acetabulum showing a metal shell cup anchored to the pelvic bone.
- Figures 4 and 5 represent a reaming tool and blade for reaming the rim of the acetabulum and
- Figure 6 represents an alignment guide for inserting screws through the metal cup shell into the pelvic bone.

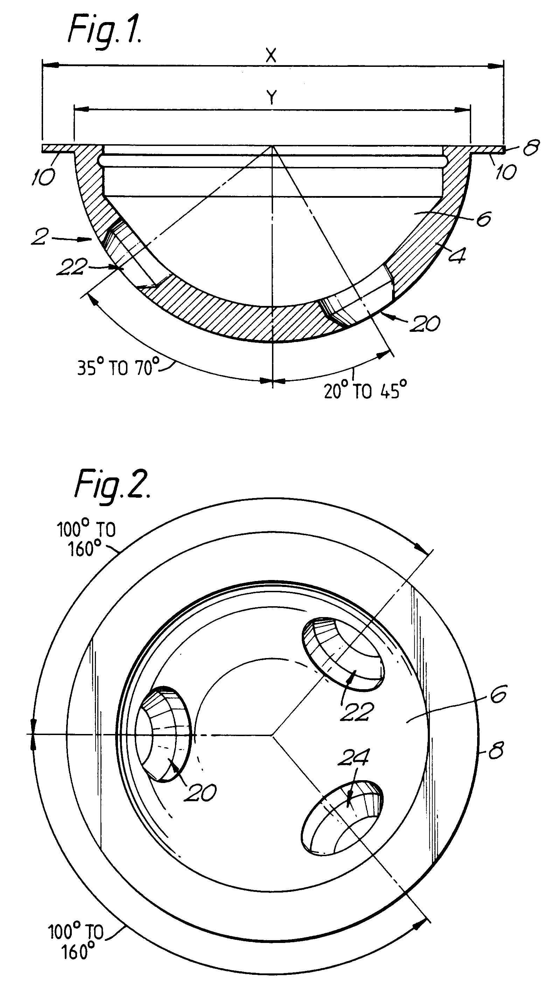

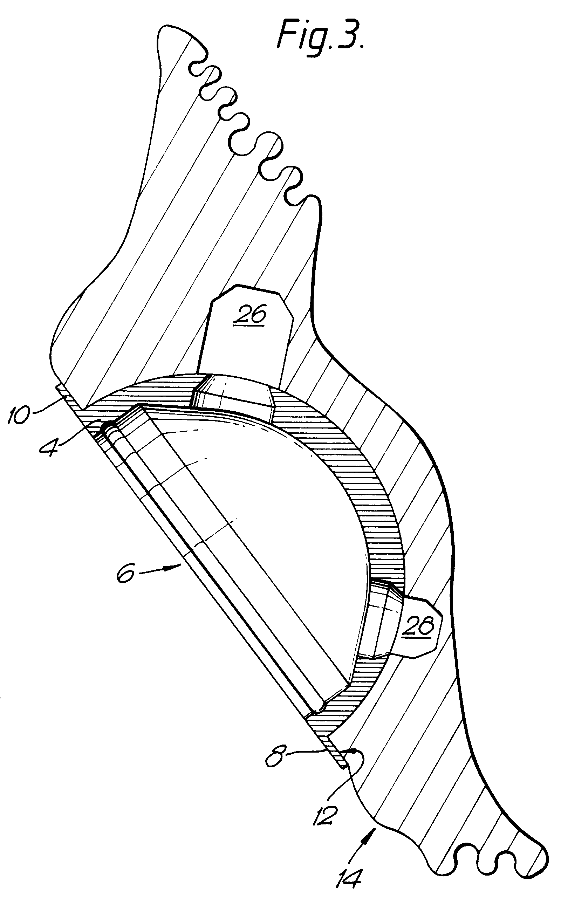

Referring to Figures 1 to 3 the metal cup shell 2 comprises a substantially hemispherical body 4 defining a bore 6. The shell includes a circumferential flange 8 having a diameter 'X' which is from 10 to 40% greater than the diameter 'Y', the widest outside diameter of the remainder of the metal cup shell. The flange 8 has a bearing surface 10 which bears against the required surface 12 of the rim of the acetabulum when the cup shell is fitted (see Figure 3). The cup shell is preferably fabricated from titanium or a titanium alloy including aluminium or vanadium.

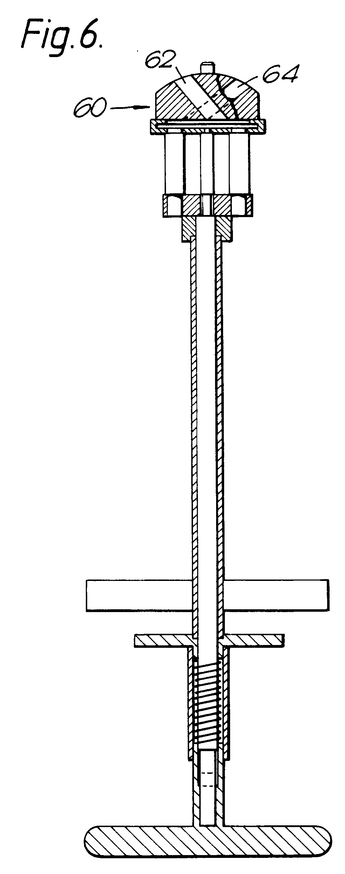

Figures 1 to 3 also illustrate the configuration of apertures and screws for optimum fixing of the metal cup shell within the pelvic bone 14 (Figure 3). The centres of apertures 22 and 24 for the inferior screws are arranged as shown in Figure 2 forming angles of from 100 to 160^{o} on either side of the aperture 20 for the superior screw. When viewed in cross-section as in Figure 1 the longitudinal axis of the aperture for the superior screw forms an angle of from 20 to 45^{o} with the longitudinal axis of the bore and those for the inferior screws form an angle of from 35 to 70^{o}. The screws 26, 28 may readily be inserted through the apertures and in correct alignment using the guide shown in Figure 6. The guide comprises an insert 60 which is positioned within the metal cup shell and three holes having diameters comparable to the outside diameter of the screws, which bones are aligned to the desired positioning for the screws. The holes of the guide are aligned with the apertures in the metal cup shell and the screws inserted in the respective holes in the guide and driven into the pelvic bone to securely anchor the metal cup shell.

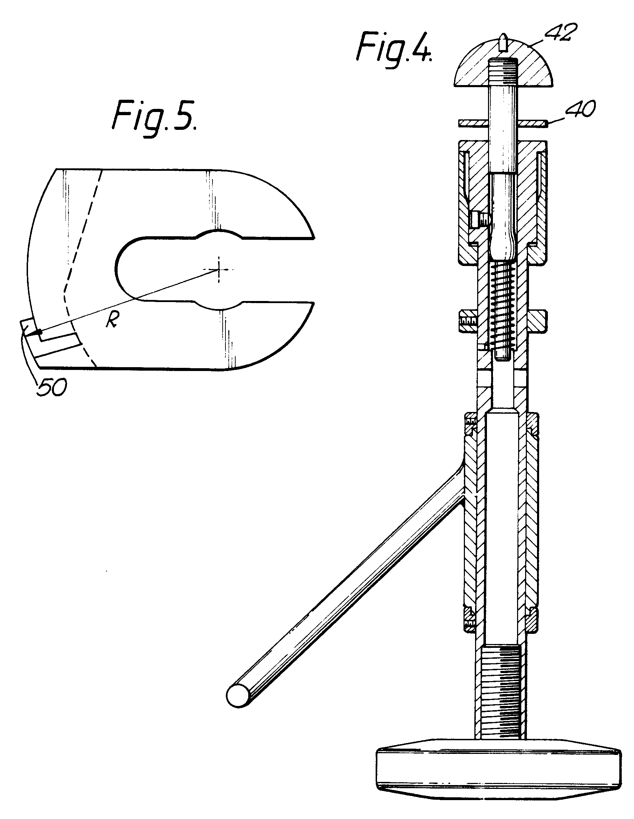

In order to ensure good fitting of the metal cup shell the acetabulum must be reamed in the conventional manner and the rim of the acetabulum must be shaved or reamed to provide a mating surface for the bearing surface of the flange. Figure 4 illustrates a reaming tool comprising a blade 40 for shaving the rim of the acetabulum. The end 42 of the tool comprises a centralising body which is inserted in the acetabulum and the instrument rotated so that the blade 40 shaves the rim of the acetabulum to provide a smooth surface.

An example of a suitable blade is shown in Figure 5 having a cutting edge 50 and a radius R which is selected to be at least one half the diameter of the flange.

The metal cup shell of the invention may be used with conventional plastics and/or ceramic inserts to provide the spherical bearing surface for the ball of the joint. The metal cup shell may also conform to the configuration of that disclosed in our corresponding British Patent Application No. 8819589 which describes an acetabular implant comprising a metal cup shell adapted for securing to the pelvic bone of a patient and a plastics insert for receiving the ball portion of a hip joint, the bore of the metal cup shell and outer surface of the plastics insert being dimensioned to allow an interference fit of the plastics insert within the bore at the body temperature of the patient in which the surface of the metal shell defining the bore is provided with one or more apertures into which the plastics material of the insert may flow when the insert is fitted to provide a mechanical interlock between the insert and cup shell thereby securing the insert against rotational and distraction forces relative to the metal cup shell. The apertures may conveniently take the form of a concentric groove near the opening of the bore and one or more radial grooves.

## Claims

1. An acetabular implant for a hip joint prosthesis comprising a metal cup shell (2) adapted for securing in the acetabulum of a patient, the metal cup shell having a bore (6) into which a liner may be inserted to provide a spherical bearing surface for the ball portion of the hip joint, whereby the metal cup shell comprises three apertures (20, 22, 24) positioned for insertion of screws into the ilium, ischium and pubic ramus of the patient and a continuous circumferential flange (8) around the opening of the bore, characterised in that the flange has an outside diameter which is at least 10% greater than the outside diameter of the remainder of the metal cup shell at its widest point, the circumferential flange providing a bearing surface (10) for contacting the rim of the acetabulum of the patient when the metal cup shell is fitted.

2. An acetabular implant as claimed in claim 1 in which the outside diameter of the flange (8) is from 10 to 40% greater than that of the remainder of the metal cup shell.

3. An acetabular implant as claimed in claim 2 in which the outside diameter of the flange (8) is from 20 to 40% greater than that of the remainder of the metal cup shell.

4. An acetabular implant as claimed in any preceding claim in which the bearing surface (10) of the flange is planar and at substantially a right angle to the longitudinal axis of the bore.

5. An acetabular implant as claimed in any one of claims 1 to 3 in which the bearing surface (10) of the flange is frusto-conical.

6. An acetabular implant as claimed in any preceding claim in which the three apertures are arranged such that:
- when viewed in plan the angle formed between a line passing through the centre of the aperture for the superior screw and the centre of the bore and a line passing through the centre of an aperture for an inferior screw and the centre of the bore is in the range 100 to 160^{o}; and
- in the side view, the angle formed between the axis of the longitudinal axis of the aperture for the superior screw and the longitudinal axis of the bore is from 20 to 45^{o} and the angle formed between the longitudinal axis of an aperture for an inferior screw and the longitudinal axis of the bore is from 35 to 75^{o}.

7. An acetabular implant as claimed in claim 6 in which the three apertures are arranged such that:
- when viewed in plan the centres of the apertures are positioned at 0^{o}, 130^{o} and 230^{o}, the aperture for the superior screw being at 0^{o}, and
- in the side view said angle for the aperture of the superior screw is 30^{o} and said angle for the apertures of the inferior screws is 50^{o}.

8. The combination of an acetabular implant as claimed in any one of Claims 1 to 7 and one or both of:
- a reaming tool comprising a blade for shaving the rim of the acetabulum, and
- a guide tool for aligning the screws used to anchor the acetabular implant in position with the apertures (20, 22, 24) in the metal cup shell (2).

9. A combination as claimed in Claim 8 in which the blade (40) of the reaming tool has a cutting edge (50) and a radius that is at least one half the diameter of the continuous circumferential flange (8) of the acetabular implant.

10. A combination as claimed in Claim 8 or Claim 9 in which the reaming tool has a longitudinal axis and the cutting edge (50) of the blade (40) is situated in a plane substantially perpendicular to the longitudinal axis of the reaming tool.

11. A combination as claimed in any one of Claims 8 to 10 in which the guide tool comprises an insert (60) which is positioned within the metal cup shell (2), the insert (60) having three holes alignable with the apertures (20, 22, 24) of the metal cup shell (2) such that screws inserted into said holes can be driven through the apertures (20, 22, 24) into the pelvic bone to securely anchor the implant in position.

## Patentansprüche

1. Acetabularimplantat für eine Hüftgelenkprothese, mit einer Metallbecherschale (2), die angepaßt ist, um in dem Acetabulum eines Patienten befestigt zu werden, wobei die Metallbecherschale eine Aushöhlung (6) aufweist, in die eine Einlage eingesetzt werden kann, um eine sphärische Lagerfläche für den Kugelteil des Hüftgelenks zur Verfügung zu stellen, wobei die Metallbecherschale drei Öffnungen (20, 22, 24) aufweist, die zum Einsetzen von Schrauben in das Ilium, das Ischium und den Pubicramus des Patienten angeordnet sind, und einen kontinuierlichen kreisförmigen Flansch (8) um die Öffnung der Aushöhlung aufweist, dadurch gekennzeichnet, daß der Flansch einen äußeren Durchmesser aufweist, der wenigstens 10 % größer als der äußere Durchmesser des übrigen Teils der Metallbecherschale an ihrem weitesten Punkt ist, und der Ringflansch eine Stützfläche (10) zur Verfügung stellt, um den Rand des Acetabulums des Patienten zu berühren, wenn die Metallbecherschale eingepaßt ist.

2. Acetabular-Implantat nach Anspruch 1, wobei der Außendurchmesser des Flansches (8) 10 bis 40 % größer als der übrige Teil der Metallbecherschale ist.

3. Acetabular-Implantat nach Anspruch 2, wobei der Außendurchmesser des Flansches (8) 20 bis 40 % größer als der übrige Teil der Metallbecherschale ist.

4. Acetabular-Implantat nach einem der vorangegangenen Ansprüche, wobei die Stützfläche (10) des Flansches eben und im wesentlichen rechtwinklig zur Längsachse der Aushöhlung ist.

5. Acetabular-Implantat nach einem der Ansprüche 1 bis 3, wobei die Stützfläche (10) des Flansches stumpf konisch ist.

6. Acetabular-Implantat nach einem der vorangegangenen Ansprüche, wobei die drei Öffnungen so angeordnet sind, daß:
in der Draufsicht der Winkel zwischen einer Linie, die durch das Zentrum der Öffnung für die obere Schraube und das Zentrum der Aushöhlung verläuft und einer Linie, die durch das Zentrum einer Öffnung für eine untere Schraube und das Zentrum der Aushöhlung verläuft, im Bereich von 100 bis 160° ist, und
in der Seitenansicht der Winkel zwischen der Längsachse der Öffnung für die obere Schraube und der Längsachse der Aushöhlung 20 bis 45° ist und der Winkel zwischen der Längsachse einer Öffnung für eine untere Schraube und der Längsachse der Aushöhlung 35 bis 75° ist.

7. Acetabular-Implantat nach Anspruch 6, wobei die drei Öffnungen so angeordnet sind, daß:
in der Draufsicht die Zentren der Öffnungen bei 0, 130 und 230° angeordnet sind, wobei die Öffnung für die obere Schraube bei 0° liegt, und
in der Seitenansicht der Winkel für die Öffnung der oberen Schraube 30° und der Winkel für die Öffnungen der unteren Schrauben 50° ist.

8. Kombination des Acetabular-Implantats nach einem der Ansprüche 1 bis 7 mit einem oder beiden von:
einem Aufreibwerkzeug mit einem Blatt zum Schaben des Randes des Acetabulums, und
einem Führungswerkzeug zum Ausrichten der Schrauben, die zum Verankern des Acetabular-Implantats in seiner Position verwendet werden, mit den Öffnungen (20, 22, 24) in der Metallbecherschale (2).

9. Kombination nach Anspruch 8, wobei das Blatt (40) des Aufreibwerkzeugs eine Schneidkante (50) und einen Radius aufweist, der mindestens der halbe Durchmesser des kontinuierlichen Ringflansches (8) des Acetabular-Implantats ist.

10. Kombination nach Anspruch 8 oder 9, wobei das Aufreibwerkzeug eine Längsachse aufweist und die Schneidkante (50) des Blattes (40) in einer Ebene angeordnet ist, die im wesentlichen senkrecht zur Längsachse des Aufreibwerkzeuges ist.

11. Kombination nach einem der Ansprüche 8 bis 10, wobei das Führungswerkzeug einen Einsatz (60) aufweist, der innerhalb der Metallbecherschale (2) angeordnet ist, wobei der Einsatz (60) drei Löcher aufweist, die mit den Öffnungen (20, 22, 24) der Metallbecherschale (2) ausrichtbar sind, so daß in die Löcher eingesetzte Schrauben durch die Öffnungen (20, 22, 24) in den Beckenknochen geschraubt werden können, um das Implantat sicher in seiner Position zu verankern.

## Revendications

1. Implant acétabulaire pour prothèse de l'articulation de la hanche, comprenant une coquille-coupelle métallique (2) destinée à être fixée dans l'acétabulum (ou cavité cotyloïde) d'un patient, cette coquille-coupelle métallique comportant, d'une part, un évidement (6) dans lequel une garniture peut être placée de façon à offrir une surface de portée sphérique pour la partie sphérique de l'articulation de la hanche et, d'autre part, trois ouvertures (20, 22, 24), disposées de façon à permettre l'introduction de vis dans l'os iliaque, l'ischion et la branche pubienne du patient, et une collerette circonférentielle continue (8) située autour de l'entrée de l'évidement, caractérisé en ce que la collerette a un diamètre extérieur qui est supérieur d'au moins 10 % au diamètre extérieur de la partie restante de la coquille-coupelle métallique à son endroit le plus large, cette collerette circonférentielle offrant une surface de portée (10) destinée à venir au contact de la crête de l'acétabulum du patient lorsque la coquille-coupelle métallique est montée.

2. Implant acétabulaire suivant la revendication 1, selon lequel le diamètre extérieur de la collerette (8) est supérieur d'une valeur comprise entre 10 et 40 % à celui de la partie restante de la coquille-coupelle métallique.

3. Implant acétabulaire suivant la revendication 2, dans lequel le diamètre extérieur de la collerette (8) est supérieur d'une valeur comprise entre 20 et 40 % à celui de la partie restante de la coquille-coupelle métallique.

4. Implant acétabulaire suivant l'une quelconque des revendications précédentes, dans lequel la surface de portée (10) de la collerette est plane et disposée pratiquement à angle droit vis-à-vis de l'axe longitudinal de l'évidement.

5. Implant acétabulaire suivant l'une quelconque des revendications 1 à 3, dans lequel la surface de portée (10) de la collerette est tronconique.

6. Implant acétabulaire suivant l'une quelconque des revendications précédentes, selon lequel les trois ouvertures sont agencées de façon telle que :
- en vue en plan, l'angle formé par une ligne passant par le centre de l'ouverture prévue pour la vis supérieure et le centre de l'évidement et par une ligne passant par le centre d'une ouverture prévue pour une vis inférieure et le centre de l'évidement est compris entre 100 et 160° et,
- en vue de côté, l'angle formé par l'axe longitudinal de l'ouverture prévue pour la vis supérieure et par l'axe longitudinal de l'évidement est compris entre 20 et 45^{o} et l'angle formé par l'axe longitudinal d'une ouverture prévue pour une vis inférieure et par l'axe longitudinal de l'évidement est compris entre 35 et 75°.

7. Implant acétabulaire suivant la revendication 6, dans lequel les trois ouvertures sont agencées de façon telle que :
- en vue en plan, les centres des ouvertures sont disposés à 0°, 130° et 230°, l'ouverture prévue pour la vis supérieure étant située à 0°, et
- en vue de côté, l'angle correspondant à l'ouverture prévue pour la vis supérieure est égal à 30° et l'angle correspondant aux ouvertures prévues pour les vis inférieures est égal à 50°.

8. Combinaison d'un implant acétabulaire suivant l'une quelconque des revendications 1 à 7 et de l'un des deux éléments, ou des deux éléments, du groupe constitué par :
- un outil de dressage de face comprenant une lame permettant d'araser la crête de l'acétabulum et
- un outil de guidage permettant d'aligner les vis, utilisées pour ancrer l'implant acétabulaire en position, avec les ouvertures (20, 22, 24) ménagées dans la coquille-coupelle métallique (2).

9. Combinaison suivant la revendication 8, dans laquelle la lame (40) de l'outil de dressage de face comporte une arête de coupe (50) et a un rayon qui est égal au moins à la moitié du diamètre de la collerette circonférentielle continue (8) de l'implant acétabulaire.

10. Combinaison suivant l'une des revendications 8 et 9, dans laquelle l'outil de dressage de face a un axe longitudinal et l'arête de coupe (50) de la lame (40) est située dans un plan pratiquement perpendiculaire à cet axe longitudinal de l'outil de dressage de face.

11. Combinaison suivant l'une quelconque des revendications 8 à 10, dans laquelle l'outil de guidage comprend une pièce rapportée (60) qui est disposée à l'intérieur de la coquille-coupelle métallique (2), cette pièce rapportée (60) comportant trois trous pouvant être alignés avec les ouvertures (20, 22, 24) de la coquille-coupelle métallique (2) de façon telle que des vis introduites dans ces trous puissent être vissées, à travers les ouvertures (20, 22, 24), dans l'os pelvien, afin d'ancrer l'implant en position d'une manière sûre.
